# EUROPEAN PATENT APPLICATION

(11) **EP 4 371 490 A1**
(43) Date of publication of application: **22.05.2024**
(21) Application number: 22858730.9
(22) Date of filing: 17.08.2022
(51) Int. Cl.: A61B 5/346, A61B 5/308, A61B 5/327, A61B 5/00, G16H 50/20, G16H 50/70

(54) **DEEP LEARNING-BASED ELECTROCARDIOGRAM DATA NOISE REMOVAL SYSTEM**

(30) Priority: 17.08.2021 KR 20210107771
(71) Applicant: Medicalai Co., Ltd., Seoul 06302 (KR)
(72) Inventor: KWON, Joon Myoung, Seoul 06629 (KR)
(74) Representative: Isarpatent
(86) International application number: PCT/KR2022/012244
(87) International publication number: WO 2023/022493

(57) **Abstract**

Disclosed herein is a system for removing the noise of electrocardiogram data based on deep learning, the system including: an electrocardiogram measurement unit (110) configured to measure electrocardiogram data for each lead from the body of an examinee; and a style-based electrocardiogram generation unit (120) configured to extract a corresponding unique style from the electrocardiogram data measured by the electrocardiogram measurement unit (110) and convert the electrocardiogram data into and generate electrocardiogram data of a specific lead style not containing noise through the extracted unique style while reflecting the characteristics of the examinee and the characteristics of a measurement method therein through an electrocardiogram generation deep learning algorithm (121) that is constructed by being previously trained on training datasets of electrocardiogram data for each lead having less noise and the unique style of the electrocardiogram data for each lead based on a large number of pieces of electrocardiogram data.

## Description

### Technical Field

The present invention relates to a system for removing the noise of electrocardiogram data based on deep learning that can maintain the unique electrical signals of the heart from measured electrocardiogram data and effectively remove the noise caused by external electrical signals by reflecting the characteristics of an examinee and the characteristics of a measurement method therein.

### Background Art

As is well known, since the development of an electrocardiogram, knowledge related to the electrocardiogram has expanded exponentially. Through electrocardiogram tests, information about the electrical functions of the heart can be obtained, and various heart diseases such as arrhythmia, coronary artery disease, and myocardial disease can be diagnosed.

Recently, research into AI algorithms for electrocardiograms has been actively conducted. By AI algorithms, heart failure is detected, atrial fibrillation among arrhythmia rhythms is predicted, and gender is determined.

As described above, by overcoming human limitations, subtle changes in electrocardiogram waveforms can be detected and electrocardiogram interpretation can be further improved by AI algorithms.

For example, the electrocardiograms used in the medical field are 12-lead electrocardiograms. Such a 12-lead electrocardiogram is measured by attaching 10 electrodes, including 3 limb electrodes, 6 chest electrodes and 1 ground electrode, and measured electrocardiogram data can be transmitted remotely.

Meanwhile, an electrocardiogram is data output after the measurement of the electrical signals of the heart on the body surface, and is measured in the state of being mixed with considerable noise. Electromyography attributable to the contraction of muscles located in the chest and the contraction of the muscles of the limbs and the noise generated from the contact surface between the skin and electrodes are sometimes mixed with an electrocardiogram. During the taking of an electrocardiogram, the movement of the chest may be included in the electrocardiogram as the chest rises and falls while breathing. Noise is also generated in the wires extending from electrodes in contact with the body to an electrocardiogram device. Noise is also generated by the alternating current of the electrocardiogram device itself. In the case of an exercise electrocardiogram or Holter monitor electrocardiogram for which the body is moved after attachment, rather than an electrocardiogram measured in a stationary state, the noise caused by walking or movement may also be included in the electrocardiogram.

Conventionally, a method of collectively removing noise below or above a predetermined frequency is used to remove noise by collectively deleting signals below 0.05 Hz or above 150 Hz, which are frequency bands that are not mainly generated by the heart. However, there is a problem in that an electrocardiogram signal itself is deformed, and thus, the accuracy of diagnosis of disease is reduced.

As described above, it is not easy to separate and remove the noise, generated from the characteristics of an examinee, a measurement environment, and a measurement device, from electrical signals of the heart itself. The method of performing collective removal based on a predetermined frequency distorts the unique signals of an electrocardiogram and reduces the accuracy of diagnosis of disease or the accuracy of prediction of the occurrence of disease based on the electrocardiogram.

Therefore, there is a demand for technology that ensures high diagnostic accuracy by removing noise from electrocardiogram data and thus generating high-quality electrocardiograms.

### Disclosure

### Technical Problem

A technical object to be achieved by the spirit of the present invention is to provide a system for removing the noise of electrocardiogram data based on deep learning that can maintain the unique electrical signals of the heart from measured electrocardiogram data and effectively remove the noise caused by external electrical signals by reflecting the characteristics of an examinee and the characteristics of a measurement method therein.

### Technical Solution

In order to accomplish the above-described object, an embodiment of the present invention provides a system for removing the noise of electrocardiogram data based on deep learning, the system including: an electrocardiogram measurement unit configured to measure electrocardiogram data for each lead from the body of an examinee; and a style-based electrocardiogram generation unit configured to extract a corresponding unique style from the electrocardiogram data measured by the electrocardiogram measurement unit and convert the electrocardiogram data into and generate electrocardiogram data of a specific lead style not containing noise through the extracted unique style while reflecting the characteristics of the examinee and the characteristics of a measurement method therein through an electrocardiogram generation deep learning algorithm that is constructed by being previously trained on training datasets of electrocardiogram data for each lead having less noise and the unique style of the electrocardiogram data for each lead based on a large number of pieces of electrocardiogram data.

In this case, the electrocardiogram generation deep learning algorithm may include a plurality of electrocardiogram generation deep learning algorithms that are generated for respective leads and constructed by being previously trained on the training datasets.

Furthermore, the electrocardiogram generation deep learning algorithm for each lead may be trained on electrocardiogram data having one or more unique styles.

Furthermore, the electrocardiogram generation deep learning algorithm may be implemented by an autoencoder or a generative adversarial network alone or by merging an autoencoder and a generative adversarial network together, and may extract the unique style of the electrocardiogram data for each lead.

Furthermore, the autoencoder may include an encoder configured to represent a unique style of electrocardiogram data and a decoder configured to restore the unique style to the original electrocardiogram data, and may be trained on the unique style of the electrocardiogram data.

Furthermore, the generative adversarial network may include a generator configured to generate synthetic electrocardiogram data using randomly generated variables as input and a discriminator configured to discriminate whether the synthetic electrocardiogram data is similar to actual electrocardiogram data, and the generator may be trained on the unique style of the electrocardiogram data.

Moreover, the generator may be changed into a form of the autoencoder and the autoencoder and the generative adversarial network may be merged together.

### Advantageous Effects

According to the present invention, the unique electrical signals of the heart may be maintained from measured electrocardiogram data, the noise caused by external electrical signals may be effectively removed by reflecting the characteristics of an examinee and the characteristics of a measurement method therein, diseases may be more accurately predicted and diagnosed through the electrocardiogram data from which noise has been removed, and the degree of noise may also be quantified by comparing and analyzing the electrocardiogram data from which noise has been removed with original electrocardiogram data.

### Description of Drawings

FIG. 1 shows a schematic block diagram of a system for removing the noise of electrocardiogram data based on deep learning according to an embodiment of the present invention;
FIG. 2 illustrates the autoencoder of the system for removing the noise of electrocardiogram data based on deep learning shown in FIG. 1;
FIG. 3 illustrates the generative adversarial network of the system for removing the noise of electrocardiogram data based on deep learning shown in FIG. 1; and
FIG. 4 illustrates the removal of noise performed by the system for removing the noise of electrocardiogram data based on deep learning shown in FIG. 1.

### Mode for Invention

Embodiments of the present invention having the above-described features will be described in more detail below with reference to the accompanying drawings.

A system for removing the noise of electrocardiogram data based on deep learning according to an embodiment of the present invention includes: an electrocardiogram measurement unit 110 configured to measure electrocardiogram data for each lead from the body of an examinee; and a style-based electrocardiogram generation unit 120 configured to extract a corresponding unique style from the electrocardiogram data measured by the electrocardiogram measurement unit 110 and convert the electrocardiogram data into and generate electrocardiogram data of a specific lead style not containing noise through the extracted unique style while reflecting the characteristics of the examinee and the characteristics of a measurement method therein through an electrocardiogram generation deep learning algorithm 121 that is constructed by being previously trained on training datasets of electrocardiogram data for each lead having less noise and the unique style of the electrocardiogram data for each lead based on a large number of pieces of electrocardiogram data. The system for removing the noise of electrocardiogram data based on deep learning is characterized by increasing the accuracy of diagnosis and prediction of disease by generating the electrocardiogram data from which noise has been removed.

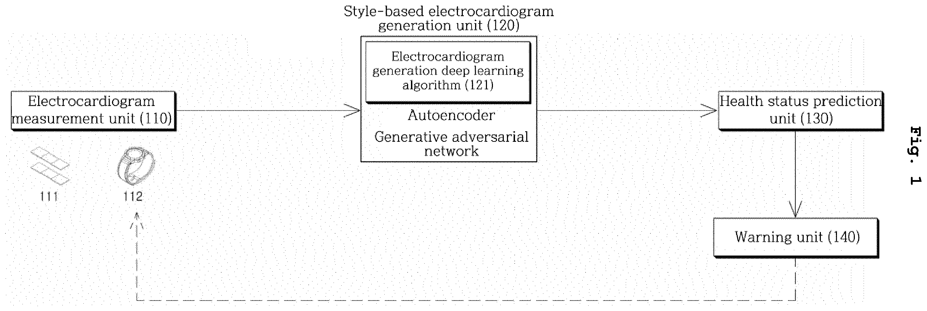

The system for removing the noise of electrocardiogram data based on deep learning configured as described above will be described in more detail below with reference to FIGS. 1 to 4, as follows.

First, the electrocardiogram measurement unit 110 measures electrocardiogram data for each of one or more leads from the body of the examinee, and provides the electrocardiogram data to the style-based electrocardiogram generation unit 120 by transmitting it.

For example, the electrocardiogram measurement unit 110 may measure asynchronous or synchronous electrocardiograms by including a wearable electrocardiogram patch 111, a smartwatch 112 or a 6-lead electrocardiogram bar for short-term measurement capable of contact or contactless electrocardiogram measurement during daily life, or an electrocardiogram device in a medical institution capable of 1- or more-lead standard lead electrocardiogram measurement.

In this case, the electrocardiogram measurement unit 110 may measure successive electrocardiograms of the examinee and transmit them to the style-based electrocardiogram generation unit 120, or may measure two electrocardiograms at different times and transmit them to the style-based electrocardiogram generation unit 120.

Next, the style-based electrocardiogram generation unit 120 converts electrocardiogram data for each lead, provided from the electrocardiogram measurement unit 110, into electrocardiogram data from which noise has been removed while reflecting the characteristics of the examinee and the characteristics of a measurement method through the electrocardiogram generation deep learning algorithm 121.

More specifically, the style-based electrocardiogram generation unit 120 may extract a corresponding unique style from the electrocardiogram data measured by the electrocardiogram measurement unit 110 and convert the electrocardiogram data into and generate electrocardiogram data of a specific lead style not containing noise through the extracted unique style while reflecting the characteristics of the examinee and the characteristics of the measurement method therein through the electrocardiogram generation deep learning algorithm 121 constructed by being previously trained on training datasets of electrocardiogram data for each lead having less noise and the unique style of the electrocardiogram data for each lead based on a large number of pieces of electrocardiogram data, such as standard 12-lead electrocardiogram data accumulated in medical institutions.

In other words, an electrocardiogram of each lead is a three-dimensional electrical flow measured in the direction (a potential vector) in which the heart is viewed from a given direction, and has its own unique style. For example, an electrocardiogram of each lead may have its own unique style as a V6 lead electrocardiogram always has a positively drawn style, and includes not only the characteristics of P, Q, R, S, and T waveforms but also curve bends or noise signals that cannot be specified with existing medical knowledge.

In this case, the electrocardiogram generation deep learning algorithm 121 extracts unique styles, each of which is the characteristic of each piece of corresponding lead electrocardiogram data, from the large-scale electrocardiogram data accumulated in medical institutions and uses them as learning datasets. A plurality of electrocardiogram generation deep learning algorithms may be generated for respective leads and constructed by being previously trained on training datasets. An electrocardiogram generation deep learning algorithm for each lead is trained on electrocardiogram data having one or more unique styles, so that the electrocardiogram generation deep learning algorithm 121 can be trained based on not only one piece of electrocardiogram data but also electrocardiogram data having two or more unique styles.

For example, in order to generate V1 lead electrocardiogram data, the electrocardiogram generation deep learning algorithm 121 may be trained on the unique styles of V1 lead electrocardiogram data based on large-scale V1 lead electrocardiogram data, and may convert the form of the V1 lead electrocardiogram data, provided from the electrocardiogram measurement unit 110, into a corresponding unique style and generate data of the style.

Meanwhile, the electrocardiogram generation deep learning algorithm 121 may identify the unique style of electrocardiogram data for each lead with high accuracy and may convert the electrocardiogram data for each lead more accurately and generate data based on the identified unique style while reflecting the characteristics of an examinee attributable to the age, gender, and disease of the examinee, etc., and the characteristics of the measurement method attributable to the attached positions of electrodes, an electrocardiogram device, etc. therein.

Furthermore, the electrocardiogram generation deep learning algorithm 121 may extract unique styles, which are potential characteristics, for given electrocardiogram data through learning using a self-supervised learning method, and may convert the electrocardiogram data, input from the electrocardiogram measurement unit 110, into electrocardiogram data of each unique style and generate the electrocardiogram data of the unique style. For example, the electrocardiogram generation deep learning algorithm 121 may be implemented by an autoencoder or a generative adversarial network alone or by merging an autoencoder and a generative adversarial network together, so that it can extract the unique style of electrocardiogram data for each lead and then generate electrocardiogram data of the corresponding unique style.

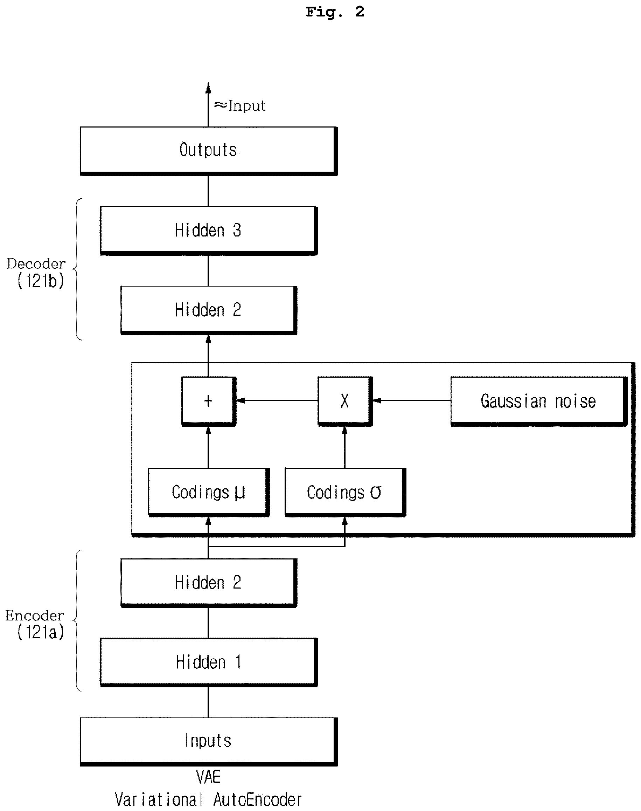

For example, referring to FIG. 2, the autoencoder includes an encoder 121a configured to represent the unique style of electrocardiogram data and a decoder 121b configured to restore the unique style to the original electrocardiogram data. The autoencoder may be trained on the unique style of the electrocardiogram data to restore the original electrocardiogram data through the process of inputting electrocardiogram data to the encoder 121a including one or more hidden layers Hidden 1 and Hidden 2, using the decoder 121b including one or more hidden layers Hidden 2 and Hidden 3 from the extracted unique style, determining the structure of the original electrocardiogram data with Gaussian noise added thereto, and restoring the electrocardiogram data used as input.

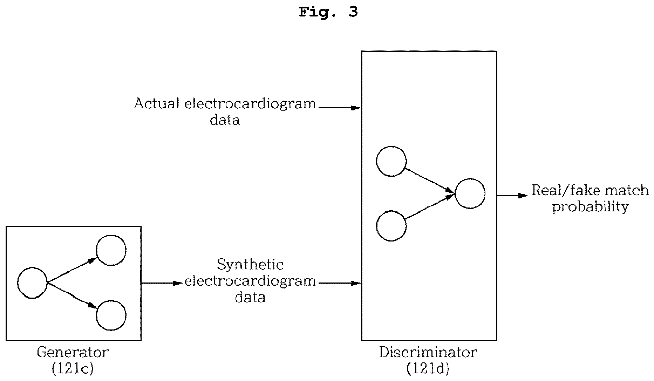

Alternatively, referring to FIG. 3, the generative adversarial network includes a generator 121c configured to generate synthetic electrocardiogram data using randomly generated variables as input, and a discriminator 121d configured to discriminate whether the synthetic electrocardiogram data is similar to actual electrocardiogram data. The generator 121c attempts to generate synthetic electrocardiogram data so that the discriminator 121d cannot discriminate between the synthetic electrocardiogram data and actual electrocardiogram data, and the generator 121c may be trained on the unique style of the electrocardiogram data in the process in which the discriminator 121d attempts to appropriately discriminate between the synthetic electrocardiogram data and the actual electrocardiogram data.

Meanwhile, the generator 121c may be changed into an autoencoder and the autoencoder and the generative adversarial network may be merged together. It may be implemented by conditionally entering an index for desired electrocardiogram data together with random variables.

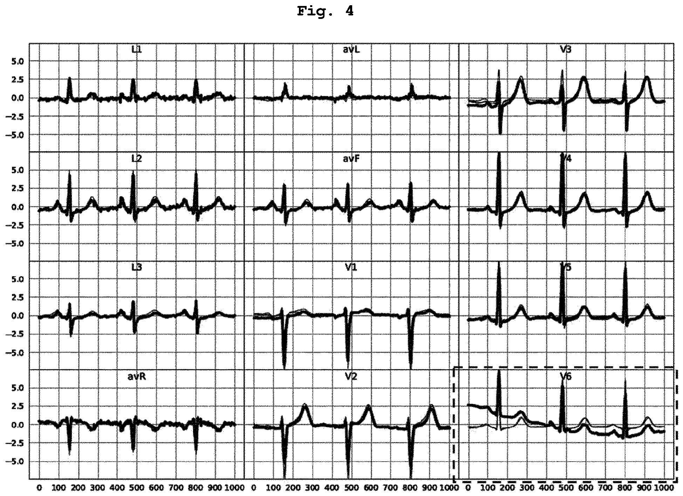

FIG. 4 illustrates the removal of noise performed by the system for removing the noise of electrocardiogram data based on deep learning shown in FIG. 1. Referring to this drawing, the removal of noise will be described in detail as follows.

The electrocardiogram data accumulated in medical institutions is electrocardiograms with the noise thereof reduced as much as possible. At actual medical sites, electrocardiogram electrodes are attached to a patient's body, observation is performed for a predetermined period of time, noise is reduced by having the patient hold their breath or relax his or her overall body, and then an electrocardiogram is taken. When a taken electrocardiogram has a lot of noise and is not valuable as a medical record, the corresponding electrocardiogram is deleted and an electrocardiogram is taken again. The electrocardiogram data for the training of the electrocardiogram generation deep learning algorithm 121 may be electrocardiograms having less noise.

Accordingly, when electrocardiogram data having a lot of noise measured by the electrocardiogram measurement unit 110 is input to the electrocardiogram generation deep learning algorithm 121, it may be converted into electrocardiogram data of a unique style having less noise and then output.

Furthermore, by applying noise filtering customized for each examinee while reflecting the characteristics of the examinee and the characteristics of the measurement method therein, the distortion of an electrocardiogram signal may be removed, and only noise may be removed while leaving the inherent tremor of the heart.

FIG. 4 is a graph illustrating the results of experiments using the electrocardiogram generation deep learning algorithm 121. The thick lines represent the actual electrocardiograms acquired by the electrocardiogram measurement unit 110, and the thin lines represent the results of the generation of electrocardiograms using the electrocardiogram generation deep learning algorithm 121.

In the example of the V6 lead in the lower right corner, the baseline was actually moved due to noise. It can be seen that noise was removed in the process of generating V6 lead data by inputting the above data into the electrocardiogram generation deep learning algorithm 121.

As described above, the type of electrocardiogram information input to the electrocardiogram generation deep learning algorithm 121 is identified by identifying the characteristic of each electrocardiogram. This may be used to generate new electrocardiogram information.

Furthermore, synchronized electrocardiogram information may be generated. However, when the model used for the diagnosis of disease uses asynchronous electrocardiograms or is a model that does not use synchronized time series information, it may be possible to generate asynchronous electrocardiogram information or generate electrocardiogram information without considering synchronization.

In addition, when an attempt is made to predict disease using electrocardiogram data, it is important to measure the noise included in input electrocardiogram data. Through this, whether to use the electrocardiogram to predict disease or to re-measure it may be determined, and the reliability of the results output after the input of electrocardiogram data may be checked in advance.

Therefore, as a method of measuring noise in electrocardiogram data, the degree of noise in the electrocardiogram may be measured by comparing the initially input electrocardiogram data with the electrocardiogram data from which the noise has been removed.

Furthermore, when electrocardiogram data cannot be measured due to reasons such as the reason that some leads or sections of electrocardiogram data contain a lot of noise or the reason that electrode contact is lost, electrocardiogram data of noise-free leads is generated. By filling in missing electrocardiogram data, the more accurate diagnosis, prediction, and examination of disease may be performed.

Furthermore, through a health status prediction unit 130, electrocardiogram data which is generated by the style-based electrocardiogram generation unit 120 and from which noise has been removed is used as input, so that the diseases that can be predicted from electrocardiogram data can be more accurately predicted.

For example, the health status prediction unit 130 may diagnose and predict diseases of the circulatory system, endocrine, nutritional and metabolic diseases, neoplastic diseases, mental and behavioral disorders, diseases of the nervous system, diseases of the eyes and their appendages, ear and mastoid diseases, diseases of the respiratory system, diseases of the digestive system, diseases of the skin and skin tissue, diseases of the musculoskeletal system and compressive tissue, diseases of the genitourinary system, pregnancy, childbirth and postpartum diseases, and congenital malformations, deformities, and chromosomal abnormalities.

In addition, through the health status prediction unit 130, the damage caused by physical trauma may be identified, the prognosis may be checked, pain may be measured, the risk of death or worsening attributable to trauma may be predicted, concurrent complications may be detected or predicted, specific conditions that appear before and after birth may be detected or predicted, and, as a healthcare area, the health status of an examinee, which may lead to services such as aging, sleep, weight, blood pressure, blood sugar, oxygen saturation, metabolism, stress, tension, fear, drinking, smoking, problematic behavior, lung capacity, the amount of exercise, pain management, obesity, body mass, body composition, diet, exercise type, and lifestyle recommendation, emergency management, chronic disease management, medication prescription, test recommendation, and checkup recommendation, nursing, telehealth management, telemedicine, vaccination post-vaccination management, etc., may be measured, diagnosed, examined, and predicted.

Meanwhile, the health status prediction unit 130 may generate the generalized base electrocardiogram of the normal healthy status of an examinee, may then perform monitoring to check whether an electrocardiogram is measured without error and whether there are any abnormalities in the health status of the examinee by comparing and analyzing the generalized base electrocardiogram with the electrocardiogram data provided in real time from the electrocardiogram measurement unit 110, and, when an error or abnormality is predicted, may generate warning information through a warning unit 140 and transmit warning information together with a beep sound through the single-lead electrocardiogram measurement unit 110 in the form of a smartwatch or a separate smart device.

Accordingly, according to the configuration of the system for removing the noise of electrocardiogram data based on deep learning described above, the unique electrical signals of the heart may be maintained from measured electrocardiogram data, the noise caused by external electrical signals may be effectively removed by reflecting the characteristics of an examinee and the characteristics of a measurement method therein, diseases may be more accurately predicted and diagnosed through the electrocardiogram data from which noise has been removed, and the degree of noise may also be quantified by comparing and analyzing the electrocardiogram data from which noise has been removed with original electrocardiogram data.

The embodiments described in the present specification and the configurations shown in the drawings are only the most preferred embodiments of the present invention and do not represent the overall technical spirit of the present invention. Accordingly, it should be understood that at the time when the present application is filed, there may be various equivalents and modifications that can replace the above embodiments and/or the configurations.
110: electrocardiogram measurement unit 120: style-based electrocardiogram generation unit
121: electrocardiogram generation deep learning algorithm 121a: encoder
121b: decoder 121c: generator
121d: discriminator 130: health status prediction unit
140: warning unit

## Claims

1. A system for removing noise of electrocardiogram data based on deep learning, the system comprising:
an electrocardiogram measurement unit configured to measure electrocardiogram data for each lead from a body of an examinee; and
a style-based electrocardiogram generation unit configured to extract a corresponding unique style while reflecting characteristics of the examinee and characteristics of a measurement method therein from the electrocardiogram data measured by the electrocardiogram measurement unit through an preconstructed electrocardiogram generation deep learning algorithm, wherein the electrocardiogram generation deep learning algorithm is preconstructed by being previously trained on training datasets including an electrocardiogram data having less noise for each lead and a unique style of the electrocardiogram data for each lead based on a large number of pieces of electrocardiogram data,
and generate the electrocardiogram data of a specific lead style not containing noise by converting the electrocardiogram data through the extracted unique style.

2. The system of claim 1, wherein the electrocardiogram generation deep learning algorithm is preconstructed by being previously trained on the training datasets plurally generated for respective leads of the electrocardiogram data..

3. The system of claim 2, wherein the electrocardiogram generation deep learning algorithm for each lead is trained on electrocardiogram data having one or more unique styles.

4. The system of claim 1, wherein the electrocardiogram generation deep learning algorithm is implemented by an autoencoder or a generative adversarial network alone or by merging an autoencoder and a generative adversarial network together, and extracts the unique style of the electrocardiogram data for each lead.

5. The system of claim 4, wherein the autoencoder includes an encoder configured to represent a unique style of electrocardiogram data and a decoder configured to restore the unique style to the original electrocardiogram data, and is trained on the unique style of the electrocardiogram data.

6. The system of claim 4, wherein the generative adversarial network includes a generator configured to generate synthetic electrocardiogram data using randomly generated variables as input and a discriminator configured to discriminate whether the synthetic electrocardiogram data is similar to actual electrocardiogram data, and the generator is trained on the unique style of the electrocardiogram data.

7. The system of claim 5 or 6, wherein the generator is changed into a form of the autoencoder and the autoencoder and the generative adversarial network are merged together.
